# EUROPEAN PATENT SPECIFICATION

(11) **EP 1 606 042 B1**
(45) Date of publication and mention of the grant of the patent: **22.12.2010**
(21) Application number: 04749379.6
(22) Date of filing: 12.03.2004
(51) Int. Cl.: B01D 53/88, B01D 53/22, B01J 20/02

(54) **FILTERING DEVICE INCORPORATING NANOPARTICLES**
FILTERVORRICHTUNG MIT NANOTEILCHEN
DISPOSITIF DE FILTRAGE CONTENANT DES NANOPARTICULES

(30) Priority: 13.03.2003 US 387854
(43) Date of publication of application: 21.12.2005
(60) Divisional application: 10011430.5
(73) Proprietor: Applied Nanoscience Inc., Hauppauge NY 11788 (US)
(72) Inventor: Applied Nanoscience Inc., Hauppauge NY 11788 (US)
(74) Representative: Liesegang, Eva
(86) International application number: PCT/US2004/007822
(87) International publication number: WO 2004/098753

(56) References cited:
- US-A1- 2001 000 067
- US-A1- 2002 023 419
- US-A1- 2002 035 032

## Description

### TECHNICAL FIELD

This invention relates to a filtering device for removing biological contaminants such as bacteria, fungi, viruses, and toxins from nonaqueous fluids.

### BACKGROUND ART

A number of patents exist with devices employing both hydrophobic and hydrophilic filters. United States patent no. 6,375,854 and copending patent application serial no. 10/128,367, filed on April 22, 2002, are notable examples.

Furthermore, United States patent application 20020035032, published on March 21, 2002, discloses metal oxide and metal hydroxide nanocrystals (also termed "nanoparticles") which can be used in the form of powder or pellets for destroying bacteria, fungi, viruses, and toxins. According to that patent application, preferred metal oxides and hydroxides include MgO, CeO₂, AgO, SrO, BaO, CaO, TiO₂, ZrO₂, FeO, V₂O₃, V₂O₅, Mn₂O₃, Fe₂O₃, NiO, CuO, Al₂O₃, SiO₂, ZnO, Ag₂O, Mg(OH)₂, Ca(OH)₂, Al(OH)₃, Sr(OH)₂, Ba(OH)₂, Fe(OH)₃, Cu(OH)₃, Ni(OH)₂; Co(OH)₂, Zn(OH)₂, Ag(OH), and mixtures thereof.

That application indicates the nanoparticles can be used alone or can have at least a portion of their surfaces coated with either (a) a second metal oxide different from the first metal oxide and selected from oxides of metals selected from the group consisting of Ti, V, Fe, Cu, Ni, Co, Mn, Zn, Al, Ce, Sr, Ba, and mixtures thereof or (b) metal nitrates such as those selected from the group consisting of Cu(NO₃)₂, Ce(NO₃)₃, AgNO₃, and mixtures thereof. In a preferred embodiment, TiO₂ is coated with a mixture of cerium nitrate and copper nitrate to form [Ce(NO₃)₃--Cu(NO₃)₃]TiO₂.

Another embodiment of that application has reactive atoms stabilized on the surfaces of particulate metal oxides; such reactive atoms are different from the atoms forming the metal oxide. Again the oxides are selected from the group consisting of MgO, CeO₂, AgO, SrO, BaO, CaO, TiO₂, ZrO₂, FeO, V₂O₃, V₂O₅, Mn₂O₃, Fe₂O₃, NiO, CuO, Al₂O₃, SiO₂, ZnO, Ag₂O, and mixtures thereof. Preferably, the reactive atoms are selected from the group consisting of halogens and Group I metals. When halogens are the reactive atoms being stabilized on the surfaces of the particles, the atoms can be atoms of the same halogen, e.g., only chlorine atoms, or mixtures of atoms of different halogens, e.g., chlorine and bromine atoms.

And a final embodiment of that application has particulate metal oxides having species different from the metal oxide adsorbed on the surfaces of the metal oxide. Once more the oxides are selected from the group consisting of MgO, CeO₂, AgO, SrO, BaO, CaO, TiO₂, ZrO₂, FeO, V₂O₃, V₂O₅, Mn₂O₃, Fe₂O₃, NiO, CuO, Al₂O₃, SiO₂, ZnO, Ag₂O, and mixtures thereof. Preferably, the adsorbed species are selected from the group consisting of oxides of Group V elements, oxides of Group VI elements, and ozone. Preferred oxides of Group V and VI elements are NO₂ and SO₂, respectively.

United States patent application 20020070172, published on June 13, 2002, discloses the use of particle, pellets, and granules of fine-particle or nanoparticle iron oxides and/or iron oxyhydroxides to remove pollutants in a unit through which a fluid flows. In water purification the material is used in horizontal- or vertical-flow filters or adsorber columns or added to the water. In gas purification it is used in adsorbers for binding undesirable components such as hydrogen sulfide mercaptans, and hydrogen cyanaide as well as other phosphorus, arsenic, antimony, sufur, selenium, tellurium, cyano, and heavy metal compounds in waste gases. Gases such as HF, HCl, H₂s, SOₓ, and NOₓ can also be adsorbed.

US 2002/0023419 describes a filter for filtering gases which comprises a composite material based on at least one open-structured and material-permeable support and having at least one inorganic component which comprises essentially at least one compound of a metal, semimetal or a mixed metal with at least one element of main groups III to VII.

Finally, in June, 2002, the Subcommittee on Nanoscale Science, Engineering and Technology of the Committee on Technology for the National Science and Technology Council published the *National Nanotechnology Initiative: the Initiative and Its Implementation Plan* as a detailed technical report associated with the Supplemental Report to the President's FY 2003 Budget. This report, on pages 66 and 67, states:
"Gas mask filters used in nuclear, biological, and chemical (NBC) applications remove toxic chemical by a process that remains essentially a WWII technology. The material responsible for chemical vapor/gas removal is an activated carbon impregnated using a Whetlerite method that impregnates metal oxides, such as, copper, zinc, molybdenum, and silver, into the larger pores of the carbon. In a very real sense activated carbon is replete with nanopores ranging from about 0.5 nm to 500 nm. Nanoscience can provide new opportunities for high surface area adsorbents and can further provide new molecular templating techniques that can augment the bonding strength. Optimized in another way, nanoporous materials can assist in the separation technologies necessary to geometrically block the migration of agents through use of a membrane.

"Collective protection systems and and protective clothing frequently utilize fibrous filters to remove agents. High-efficiency particulate arresting (HEPA) filters can be effective against particulates; even the biological toxins that might be dispersed as aerosols could be filtered out by HEPA. The use of nanotubes, nanofilaments, and nanoporous membranes might make these filters even more effective, and might include catalytic degraders as well."

None of the preceding, however, suggests using nanoparticles that are known to be capable of destroying bacteria, fungi, viruses, or toxins in conjunction with hydrophobic or hydrophilic filters. Nor, although the article seems to suggest using nanoparticles, themselves, to create a filter and may indicate impregnating carbon with nanoparticles, do the preceding seem to suggest coating any type of filter with nanoparticles, placing nanoparticle pellets adjacent to any type of filter, or impregnating any filter material other than carbon with nanoparticles.

### DISCLOSURE OF INVENTION

The present invention, in a first embodiment, combines any type of nanoparticle that is known to be capable of destroying bacteria, fungi, viruses, or toxins with one or more hydrophobic or hydrophilic filters.

The nanoparticles can be in the form of either a powder or a pellet, wherein the powder form does not form part of the invention.

When a powder is employed, not forming part of the present invention, the hydrophobic or hydrophilic filter is, using any technique that is known in the art, either coated or impregnated with the powder.

Preferably, in the case of coating, not forming part of the present invention, the hydrophobic or hydrophilic filter carries an electrostatic charge of a given polarity; and the nanoparticles are, using any technique that is well known in the art, given a charge of opposite polarity, either in the creation of the nanoparticle or through electrical induction.

In an article copyrighted by the American Chemical Society (Langmuir 2002, 18, 6679-6686) and entitled "Metal Oxide Nanoparticles as Bactericidal Agents" Peter K. Stoimenov, Rosalyn L. Klinger, George L Marchin, and Kenneth J. Klabunde, for example, explain "... all AP-MgO/X₂ formulations are positively charged (27.0 mV (AP-MgO/Br₂), 33.0 mV (AP-MgO/Cl₂), and 352 mV (AP-MgO) at 0.01 ionic strength NaCl)." (According to that article, "AP" indicates that the nanoparticle has been prepared through an aerogel procedure.)

The present invention utilizes pellets, such pellets are placed adjacent to a hydrophobic or hydrophilic filter and, together with the filter, are contained within an encasement having an inlet and an outlet

Preferably one or more hydrophobic filters are utilized in serial fluid communication with one or more hydrophilic filters. The nanoparticle coating, not forming part of the present invention, or the pellets of nanoparticles can be placed on either the upstream or the downstream side of any one or more hydrophobic or hydrophilic filters. The filters are contained within an encasement having an inlet and an outlet, whether one or more filters is coated, not forming part of the present invention, or has pellets adjacent to such filter or filters.

In the embodiment wherein the pellets are placed on a side of a filter which has no other filter facing it, some means for containing the pellets is necessary. In the case of the powder used to coat the filter, not forming part of the present invention, (rather than being impregnated into the filter), not forming part of the present invention, a containment means is merely preferable.

For the pellets, it is preferable to have the inlet or the outlet (depending upon which is closer to the nanoparticles) of the encasement consist of one or more apertures having a maximum dimension that is less than the minimum dimension of the pellets.

For the powder coating, not forming part of the present invention, a membrane having a pore size smaller than the powder particles but large enough not to impede the flow of a gas substantially, preferably a pore size at least as large as the pore size of the hydrophobic or hydrophilic filter having the smallest pore size, is preferably placed across the inlet or outlet (depending upon which is closer to the nanoparticles).

Such a membrane may similar be used when the hydrophobic or hydrophilic filter is impregnated with nanoparticles, not forming part of the present invention, although this is not generally done.

In further embodiments, the present invention utilizes, in place of the hydrophobic or hydrophilic filter, a filter of any type of known filter material except, carbon.

### BRIEF DESCRIPTION OF DRAWINGS

FIG. 1 portrays, in a cutaway view, nanoparticles adjacent to a filter, where the size and number of the nanoparticles has been varied for purposes of illustration.
FIG. 2, not forming part of the present invention, illustrates, in a cutaway view, a filter coated with nanoparticles, where the thickness of the coating has been exaggerated for purposes of illustration
FIG. 3, not forming part of the present invention, shows, in a cutaway view, a filter impregnated with nanoparticles, where the size and number of the nanoparticles has been varied for purposes of illustration.
FIG. 4 depicts, in a cutaway view, an encasement having nanoparticles adjacent to and between two filters, where the size and number of the nanoparticles has been varied for purposes of illustration.
FIG. 5 is a cutaway illustration of an encasement having nanoparticles adjacent to a filter and between the filter and an inlet of the encasement, where the size and number of the nanoparticles has been varied for purposes of illustration.
FIG. 6, not forming part of the present invention, represents, in a cutaway view, an encasement having nanoparticles coating the side of a filter which is closer than any other side of any other filter to an inlet of the encasement, where the thickness of the coating has been exaggerated for purposes of illustration.
FIG. 7, not forming part of the present invention, is a cutaway view of an encasement having a filter impregnated with nanoparticles, where the size and number of the nanoparticles has been varied for purposes of illustration.

### MODES FOR CARRYING OUT THE INVENTION

As discussed above, a number of type of nanoparticles (1) are known to be capable of destroying bacteria, fungi, viruses, or toxins. The present invention combines any type of such nanoparticles (1) with one or more filters (2).

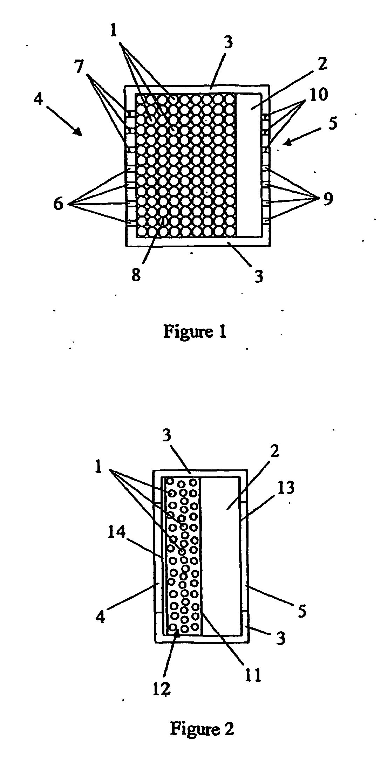

In a first principal embodiment, shown in FIG. 1, any type of nanoparticle pellets (1) that are known to be capable of destroying bacteria, fungi, viruses, or toxins are adjacent to a filter (2) within an encasement (3) having an inlet (4) and an outlet (5). Of course, if the nanoparticle pellets (1) are between the inlet (4) and the filter (2), some means must exist to contain the nanoparticle pellets (1). Any such means known in the art may be employed. Preferably, however, the one or more apertures (6) which comprise the inlet (4) each have a maximum cross-sectional dimension (7) that is less than the minimum dimension (8) of the nanoparticle pellets (1). Similarly, when the nanoparticle pellets (1) are between the outlet (5) and the filter (2), there must be a containment means, which preferably comprises having the one or more apertures (9) which comprise the outlet (5) each have a maximum dimension (10) that is less than the minimum dimension (8) of the nanoparticle pellets (1). Preferably, the nanoparticle pellets (1) are between the inlet (4) and the filter (2).

Preferably, the filter (2) has an electrical charge that is the same as the electrical charge of at least one target particle, wherein the term "target particle," as used herein, means the basic unit of any entity which the filter (2) is intended to exclude, such as a bacterium.

Optionally, the filter (2) is hydrophobic. In another optional embodiment, the filter (2) is hydrophilic.

A second principal embodiment, not forming part of the present invention, portrayed in FIG. 2, comprises a filter (2) coated on at least a first side (11) with a powder (12) of any type of nanoparticles (1) that are known to be capable of destroying bacteria, fungi, viruses, or toxins.

Preferably, coating is accomplished by having the filter (2) carry an electrical charge that is opposite to an electrical charge carried by the nanoparticles (1) in the powder (12). Also preferably, the filter (2) has an electrical charge that is the same as the electrical charge of at least one target particle.

Most preferably, an electrical charge on the filter (2) is both opposite to an electrical charge carried by the nanoparticles (1) in the powder (12) and the same as the electrical charge of at least one target particle. For example, the nanoparticle (1) can be AP-MgO/Br₂, AP-MgO/Cl₂, or AP-MgO, all of which are, as indicated above, positively charged. The filter (2) is then selected to have a negative electrical charge, which attracts the positively charged nanoparticles (1). Since, according to pages 6681 through 6682 in the *Langmuir* article quoted above, "... it is a well-established fact in the literature [citing Busscher, H. J.; Bos, R; van der Mei, H. C.; Handley, P. S. in Physical Chemistry of Biological Interfaces, Baszkin, A., Norde, W., Eds.; Marcel Dekker: New York, 2000.] that the overall charge of the bacteria and spore cells at biological pH values is negative, because of the excess number of carboxylic and other groups which upon dissociation make the cell surface negative." Thus, in this most preferred situation, the electrical charge of the filter (2) tends to repel the bacteria while any bacteria that do reach the coating nanoparticle powder (12) tend to be attracted to and destroyed by the positively charged nanoparticles (1).

Again, optionally, not forming part of the present invention, the filter (2) can be hydrophobic; and, optionally, it can be hydrophilic. An example of a commercially available hydrophobic filter is that sold under the trademarked name FILTRATE by the 3M company of St. Paul, Minnesota. And an example of a commercially available hydrophilic filter is that sold under the name Heat and Moisture Exchange Media also by the 3M company of St. Paul, Minnesota.

Also optionally, not forming part of the present invention, the filter (2) is contained within an encasement (3) having an inlet (4) and an outlet (5). Preferably, the first side (11) of the filter (2) is directed toward the inlet (4) and a second side (13) of the filter (2) is directed toward the outlet (5). And preferably, if a coated side (11), (13) of the filter (2) is directed toward the inlet (4), such inlet (4) is covered by a membrane (14) having a pore size smaller than the nanoparticles (1) but large enough not to impede the flow of a gas substantially, preferably a pore size at least as large as the pore size of the filter (2). Similarly, preferably, if a coated side (11), (13) of the filter (2) is directed toward the outlet (5), such outlet (5) is covered by a membrane (14) having a pore size smaller than the nanoparticles (1) but large enough not to impede the flow of a gas substantially, preferably a pore size at least as large as the pore size of the filter (2).

Suitable membranes (14) are termed "webbing" and are, for example, commercially available from either the 3M company of St. Paul, Minnesota, or the Vernal company of Los Angeles, California.

This principal embodiment was used so test the effectiveness of the nanoparticles (1) in destroying a bacterium when placed upon a hydrophobic filter (2).

### Example (Reference)

A portion of a top surface of each of six horizontally oriented negatively charged hydrophobic FILTRETE filters was coated with positively charged AP-MgO/Cl₂. Also on top of the filters but not necessarily just in the location of the nanoparticles were placed an average of 226,000 colony-forming units of bacterium thuringiensis. There was no flow of air through the filter.

As a control, on a portion of a top surface of each of six uncoated horizontally oriented negatively charged hydrophobic FILTRETE filters were placed an average of 226,000 colony-forming units of bacterium thuringiensis.

After twenty-four hours, the number of colony forming units on the uncoated filters had increased by an average of more than 6507 percent while the number of colony forming units on the coated filters had decreased by an average of 21.7 percent.

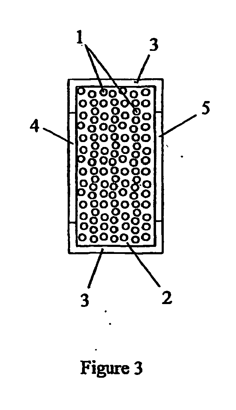

For the third principal embodiment, depicted in FIG. 3, not forming part of the present invention, a filter (2) is, using any technique that is known in the art, impregnated with any type of nanoparticles (1) that are known to be capable of destroying bacteria, fungi, viruses, or toxins.

Preferably, the filter (2) carries an electrical charge that is opposite to an electrical charge carried by the nanoparticles (1). Also preferably, the filter (2) has an electrical charge that is the same as the electrical charge of at least one target particle.

Most preferably, an electrical charge on the filter (2) is both opposite to an electrical charge carried by the nanoparticles (1) and the same as the electrical charge of at least one target particle.

Once again, optionally, the filter (2) can be hydrophobic; and, optionally, it can be hydrophilic.

Also optionally, the filter (2) is contained within an encasement (3) having an inlet (4) and an outlet (5).

The final four principal embodiments all employ an encasement (3) having an inlet (4) and an outlet (5) and containing two or more filters (2) in serial fluid communication with each other. Optionally, at least one of the filters (2) is hydrophobic; and, also optionally, at least one of the filters (2) is hydrophilic. Furthermore, preferably at least one of the filters (2) has an electrical charge that is the same as an electrical charge of at least one target particle; and, preferably, the filter (2) nearest the inlet (4) is hydrophobic.

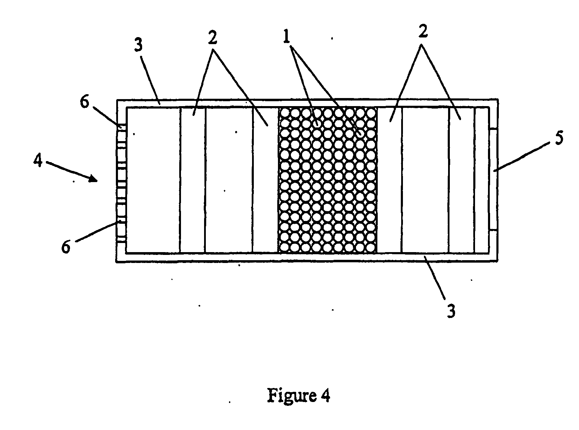

The fourth principal embodiment, illustrated in FIG. 4, has adjacent to and between at least two consecutive filters (2) any type of nanoparticle pellets (1) that are known to be capable of destroying bacteria, fungi, viruses, or toxins.

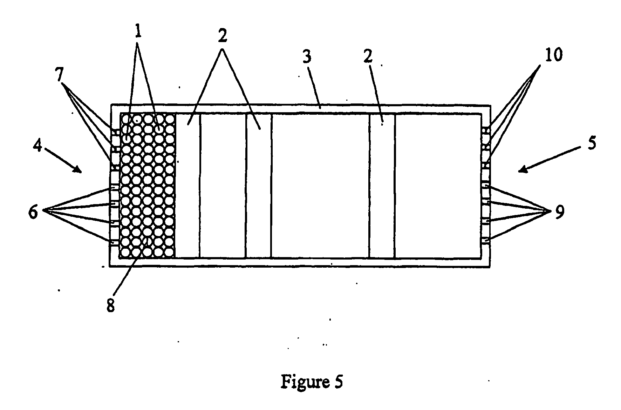

In the fifth principal embodiment, seen in FIG. 5, any type of nanoparticle pellets (1) that are known to be capable of destroying bacteria, fungi, viruses, or toxins are at least adjacent to a filter (2) that has no other filter (2) between such filter (2) and an external passageway (4), (5). As used herein, the term "external passageway" shall include both an inlet (4) and an outlet (5) and, when used in the singular, shall designate either an inlet (4) or an outlet (5). The nanoparticle pellets are between such filter (2) and the external passageway (4), (5) which is nearer to the filter (2). Preferably, such external passageway (4), (5) is the inlet (4) of the encasement (3).

Of course, as with the first principal embodiment, in the fifth principal embodiment some means must exist to contain the nanoparticle pellets (1). Any such means known in the art may be employed. Preferably, however, when the nanoparticle pellets (1) are between the filter (2) and the inlet (4), the one or more apertures (6) which comprise the inlet (4) each have a maximum dimension (7) that is less than the minimum dimension (8) of the nanoparticle pellets (1). Similarly, when the nanoparticle pellets (1) are between the outlet (5) and the filter (2), the containment means preferably comprises having the one or more apertures (9) which comprise the outlet (5) each have a maximum dimension (10) that is less than the minimum dimension (8) of the nanoparticle pellets (1).

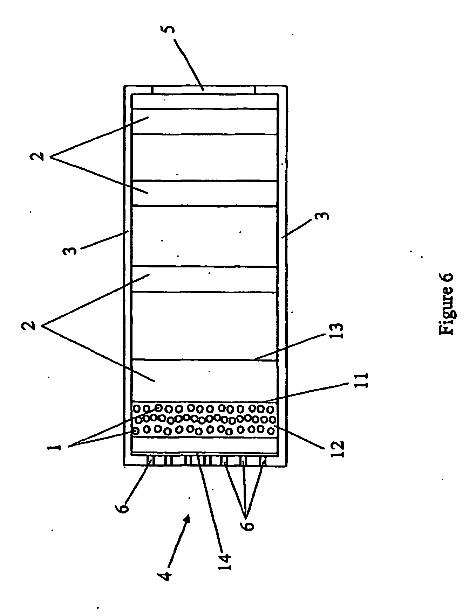

For the sixth principal embodiment, pictured in FIG. 6, not forming part of the present invention, a first side (11) of at least one filter (2) is coated with a powder (12) of any type of nanoparticles (1) that are known to be capable of destroying bacteria, fungi, viruses, or toxins.

Preferably, coating is accomplished by having the filter (2) carry an electrical charge that is opposite to an electrical charge carried by the nanoparticles (1) in the powder (12). Most preferably, an electrical charge on the filter (2) is both opposite to an electrical charge carried by the nanoparticles (1) and the same as the electrical charge of at least one target particle.

Also preferably, at least one such coated filter (2) has no other filter (2) between such filter (2) and the inlet (4) of the encasement (3); and most preferably the first side (11) of such filter (2) is directed toward the inlet (4).

When a coated side (11), (13) of a filter (2) is directed toward an external passageway (4), (5) and no other filter (2) is between such coated filter (2) and the external passageway (4), (5), such external passageway is preferably covered by a membrane (14) having a pore size smaller than the nanoparticles (1) but large enough not to impede the flow of a gas substantially, preferably a pore size at least as large as the pore size of the filter (2) which has the smallest pore size.

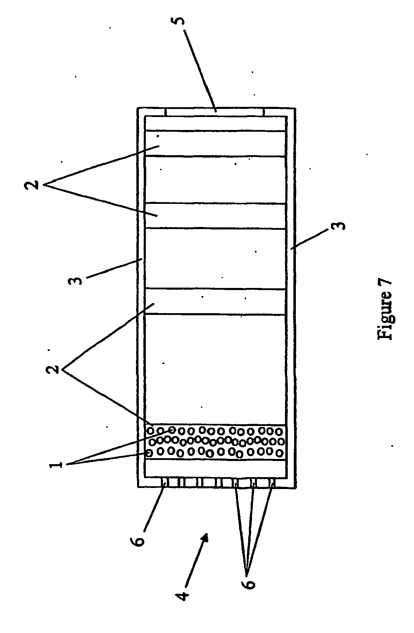

In the seventh embodiment, portrayed in FIG. 7, not forming part of the present invention, at least one filter (2), which is, preferably, the filter (2) closest to the inlet (4) of the encasement (3), is, using any technique that is known in the art, impregnated with any type of nanoparticles (1) that are known to be capable of destroying bacteria, fungi, viruses, or toxins.

Preferably, the impregnated filter (2) carries an electrical charge that is opposite to an electrical charge carried by the nanoparticles (1). Most preferably, an electrical charge on the impregnated filter (2) is both opposite to an electrical charge carried by the nanoparticles (1) and the same as the electrical charge of at least one target particle.

As used herein the term "preferable" or "preferably" means that a specified element or technique is more acceptable than another but not that such specified element or technique is a necessity.

### INDUSTRIAL APPLICABILITY

The way in which the Filtering Device Incorporating Nanoparticles is capable of exploitation in industry and the way in which the Filtering Device Incorporating Nanoparticles can be made and used are obvious from the description and the nature of the Filtering Device Incorporating Nanoparticles.

## Claims

1. A filtering device incorporating nanoparticles, which comprises:
an encasement (3) having an inlet (4) and an outlet (5);
a filter (2) within said encasement (3);
nanoparticle pellets (1) that are known to be capable of destroying bacteria, fungi, viruses, or toxins, said nanoparticle pellets (1) being adjacent to said filter (2) within said encasement (3); and
a means for containing said nanoparticle pellets (1).

2. The filtering device incorporating nanoparticles as recited in claim 1, wherein:
said filter (2) is hydrophobic.

3. The filtering device incorporating nanoparticles as recited in claim 1, wherein:
said filter (2) is hydrophilic.

4. The filtering device according to any of the foregoing claims wherein:
said filter (2) has an electrical charge that is the same as an electrical charge of at least one target particle.

5. The filtering device according to any of claims 1-4, which comprises
two or more filters (2) in serial fluid communication with each other;
wherein each inlet (4) and each outlet (5) of the encasement constitute an external passageway; and
wherein the nanoparticle pellets (1) that are known to be capable of destroying bacteria, fungi, viruses, or toxins are at least adjacent to one of said filters (2) that has no other of said filters (2) between the one of said filters (2) and an external passageway
or
wherein one of said filters (2) has no other of said filters (2) between the one of said filters (2) and an external passageway and wherein the nanoparticle pellets (1) that are known to be capable of destroying bacteria, fungi, viruses, or toxins are at least adjacent to another one of said filters (2).

6. The filtering device according to claim 5, wherein:
at least one of said filters (2) is hydrophobic.

7. The filtering device according to claim 5, wherein:
at least one of said filters (2) is hydrophilic.

8. The filtering device according to any of claims 5-7, wherein:
at least one of said filters (2) has an electrical charge that is the same as an electrical charge of at least one target particle.

9. The filtering device according to claim 8, wherein:
the one of said filters (2) that is nearest to the inlet (4) of said encasement (3) is hydrophobic.

10. The filtering device according to any of claims 5-7, wherein:
the one of said filters (2) that is nearest to the inlet (4) of said encasement (3) is hydrophobic.

## Patentansprüche

1. Filtervorrichtung, enthaltend Nanopartikel, die umfasst:
ein Gehäuse (3) mit einem Einlass (4) und einem Auslass (5);
einen Filter (2) innerhalb des Gehäuses (3);
Nanopartikel-Pellets (1), von denen bekannt ist, dass sie in der Lage sind, Bakterien, Pilze, Viren oder Toxine zu zerstören, wobei die Nanopartikel-Pellets (1) an den Filter (2) innerhalb des Gehäuses (3) angrenzen; und
Mittel zum Enthalten der Nanopartikel-Pellets (1).

2. Filtervorrichtung, enthaltend Nanopartikel, nach Anspruch 1, wobei:
der Filter (2) hydrophob ist.

3. Filtervorrichtung, enthaltend Nanopartikel, nach Anspruch 1, wobei:
der Filter (2) hydrophil ist.

4. Filtervorrichtung nach einem der vorhergehenden Ansprüche, wobei:
der Filter (2) eine elektrische Ladung hat, die dieselbe ist wie eine elektrische Ladung von wenigstens einem Zielpartikel.

5. Filtervorrichtung nach einem der Ansprüche 1-4, die umfasst:
zwei oder mehr Filter (2) in serieller Fluid-Verbindung miteinander;
wobei jeder Einlass (4) und jeder Auslass (5) des Gehäuses eine externe Verbindung darstellen;
und wobei die Nanopartikel-Pellets (1), von denen bekannt ist, dass sie in der Lage sind, Bakterien, Pilze, Viren oder Toxine zu zerstören, wenigstens an einen der Filter (2) angrenzen, der keinen anderen der Filter (2) zwischen einem der Filter (2) und einer externen Verbindung hat, oder
wobei einer der Filter (2) keinen anderen der Filter (2) zwischen einem der Filter (2) und einer externe Verbindung hat und wobei die Nanopartikel-Pellets (1), von denen bekannt ist, dass sie Bakterien, Pilze, Viren oder Toxine zerstören, wenigstens an einen anderen der Filter (2) angrenzen.

6. Filtervorrichtung nach Anspruch 5, wobei:
wenigstens einer der Filter (2) hydrophob ist.

7. Filtervorrichtung nach Anspruch 5, wobei:
wenigstens einer der Filter (2) hydrophil ist.

8. Filtervorrichtung nach einem der Ansprüche 5-7, wobei:
wenigstens einer der Filter (2) eine elektrische Ladung hat, die dieselbe wie eine elektrische Ladung von wenigstens einem Zielpartikel ist.

9. Filtervorrichtung nach Anspruch 8, wobei:
einer der Filter (2), der dem Einlass (4) des Gehäuses (3) am nächsten ist, hydrophob ist.

10. Filtervorrichtung nach einem der Ansprüche 5-7, wobei:
der eine der Filter (2), der dem Einlass (4) des Gehäuses (3) am nächsten ist, hydrophob ist.

## Revendications

1. Dispositif de filtrage incorporant des nanoparticules, qui comprend :
un boîtier (3) doté d'un orifice d'admission (4) et d'un orifice de sortie (5) ;
un filtre (2) placé à l'intérieur dudit boîtier (3) ;
des granules de nanoparticules (1) connus pour être capable de détruire des bactéries, des champignons, des virus ou des toxines, lesdits granules de nanoparticules (1) étant adjacents audit filtre (2) à l'intérieur dudit boîtier (3) ; et
des moyens pour contenir lesdits granules de nanoparticules (1).

2. Dispositif de filtrage incorporant des nanoparticules selon la revendication 1, dans lequel :
ledit filtre (2) est hydrophobe.

3. Dispositif de filtrage incorporant des nanoparticules selon la revendication 1, dans lequel :
ledit filtre (2) est hydrophile.

4. Dispositif de filtrage selon l'une quelconque des revendications précédentes, dans lequel :
ledit filtre (2) possède une charge électrique identique à la charge électrique d'au moins une particule cible.

5. Dispositif de filtrage selon l'une quelconque des revendications 1 à 4, qui comprend
deux filtres (2) ou plus en communication fluidique successive les uns avec les autres ;
dans lequel chaque orifice d'admission (4) et chaque orifice de sortie (5) du boîtier constituent un passage externe ; et
dans lequel les granules de nanoparticules (1) connus pour être capable de détruire des bactéries, des champignons, des virus ou des toxines sont au moins adjacents à l'un desdits filtres (2) qui n'a pas d'autre dit filtre (2) entre le premier desdits filtres (2) et le passage externe,
ou
dans lequel l'un desdits filtres (2) n'a pas d'autre dit filtre (2) entre l'un desdits filtres (2) et le passage externe et dans lequel les granules de nanoparticules (1) connus pour être capable de détruire des bactéries, des champignons, des virus ou des toxines sont au moins adjacents à un autre desdits filtres (2).

6. Dispositif de filtrage selon la revendication 5, dans lequel :
au moins l'un desdits filtres (2) est hydrophobe.

7. Dispositif de filtrage selon la revendication 5, dans lequel :
au moins l'un desdits filtres (2) est hydrophile.

8. Dispositif de filtrage selon l'une quelconque des revendications 5 à 7, dans lequel :
au moins l'un desdits filtres (2) possède une charge électrique identique à la charge électrique d'au moins une particule cible.

9. Dispositif de filtrage selon la revendication 8, dans lequel :
l'un desdits filtres (2) le plus proche de l'orifice d'admission (4) dudit boîtier (3) est hydrophobe.

10. Dispositif de filtrage selon l'une quelconque des revendications 5 à 7, dans lequel :
l'un desdits filtres (2) le plus proche de l'orifice d'admission (4) dudit boîtier (3) est hydrophobe.
